# EUROPEAN PATENT APPLICATION

(11) **EP 4 636 379 A1**
(43) Date of publication of application: **22.10.2025**
(21) Application number: 23903001.8
(22) Date of filing: 28.06.2023
(51) Int. Cl.: G01N 1/04, B65D 51/28, B65D 77/00, B65D 77/02, B65D 81/32, G01N 1/28, G01N 1/30

(54) **SAMPLE CONTAINER**

(30) Priority: 13.12.2022 JP 2022198610
(71) Applicant: Sansho Mec Co., Ltd., Namerikawa-shi, Toyama 936-0861 (JP)
(72) Inventor: ISSHIKI, Katsuhiko, Toyama-shi, Toyama 931-8455 (JP)
(74) Representative: Müller-Boré & Partner Patentanwälte PartG mbB
(86) International application number: PCT/JP2023/024060
(87) International publication number: WO 2024/127694

(57) **Abstract**

Provided is a sample container wherein a specimen or similar can easily be enclosed in the container in a single operation without exposure to the external environment, an unsealed state is stable, and an immersion liquid can be supplied into a storage container without delay. This sample container comprises: a storage container (A) provided with a storage apparatus (1) that retains a sample; and a reservoir container (B) provided with a reservoir apparatus (3) that stores an immersion fluid (X) in which the sample is to be immersed, and a sealing film (6) that seals an opening section of the reservoir container (3). The storage container (A) comprises an inner coupling section (3) that guides the immersion fluid (X) released from the reservoir apparatus (3) to the storage apparatus (1), and an unsealing blade (5) that faces an inner edge section of the opening section of the reservoir apparatus (3). The reservoir container (B) comprises an outer coupling section (4) that couples with the inner coupling section (3) of the storage container (A) by means of a screw pair. The screw pair comprises an impeding region that suppresses the progression of tightening immediately before the sealing film (6) and the unsealing blade (5) come into contact, and a termination region that, after the sealing film (6) and the unsealing blade (5) have come into contact, stops rotation at a rotation that is less than an intermittent angle of the unsealing blade (5). Due to tightening beyond the impeding region of the screw pair, the unsealing blade (5) of the storage apparatus (1) ruptures the sealing film (6), and communication between the reservoir apparatus (3) and the storage apparatus (1) is ensured.

## Description

### Technical Field

The present invention relates to a sample container in which an appropriate amount of immersion fluid can be supplied to a sample inside a container without exposure to the atmosphere outside the container.

### Background Art

When diagnosing a disease, a portion of the cells or tissue of a living body are collected using a biopsy mechanism, a biopsy needle, etc. The collected specimen needs to be stored and transported without damage until supplied for histopathological examination. To do that, the specimen must be enclosed in an airtight container and immersed in formalin, etc.

Formalin is an aqueous solution of formaldehyde, and is harmful to living organisms because it causes a strong cross-linking reaction. Because it also has an irritating odor, if a worker is exposed to formalin and is in an environment at risk of inhaling it, this can be harmful to the physical condition and health of the worker.

Though it is possible to install various devices to avoid problems associated with exposure to formalin, this makes immediate enclosure in a storage container at the specimen collection site extremely difficult.

To improve this situation, storage containers have been devised that can store a specimen or drug, etc., (hereafter noted as "specimen, etc.") without exposure to the outside.

Disclosed in Patent Document 1 is a container having a constitution in which a drug enclosure part and a bag are connected via a sealing part, and by opening of the sealing part, the bag and the drug enclosure part are put in communication with each other and the drug is put into the bag. Disclosed in Patent Document 2 is a container for storing a biological sample that is breakable to establish fluid communication between a first chamber, a second chamber, and a sample holder by moving a movable structure from the outside in a state with a first open end part closed by a closing body to move the sample holder and applying pressure to an easily breakable film. Disclosed in Patent Document 3 is a container assembly that includes a container for storing a tissue sample and a cover part that engages with the container, where on the cover part, there is an upper member that includes a reservoir adapted to contain a preservative, a seal that seals the reservoir, and a piercing member that is activated by pressing a spring film to break the seal.

### Prior Art Documents

### Patent Documents

Patent Document 1: Japanese Laid-Open Patent Publication No. 2019-141665
Patent Document 2: Japanese Patent No. 5211170
Patent Document 3: Japanese Patent No. 5936088

### Summary of the Invention

### Problems the Invention Is Intended to Solve

However, Patent Document 1 has the problem that a relatively difficult operation is required to open the sealing part in addition to the operation of enclosing the specimen, etc.

Patent Document 2 has the problem that though a constitution is used that establishes fluid communication between the first chamber and the second chamber by operating a flexible elastomer button or handle to break the easily breakable film, both require the complex work of placing the specimen, etc., in a sample holder that is coupled and linked to the button or handle.

In Patent Document 3, when breaking the seal, the upper member needs to be pressed from above, simultaneous with pressing the perforation or piercing member downward, while also simultaneously rotating the upper member, resulting in a complex constitution.

In this way, conventional sample containers all require different operations for enclosing the sample and supplying the preservative solution, which may lead to situations in which the operation of supplying the preservative solution is omitted or fails, etc., nullifying the effort of the specimen collection.

In either case, there is the problem that the shape and position of the rupturing section that reliably connects the chamber in which the specimen, etc., is input and the chamber in which the preservative solution is input cannot be uniformly controlled, and furthermore, there is a risk of damage to the specimen, etc., due to insufficient supply of immersion fluid, or contamination by foreign matter such as ruptured seal debris, etc.

The present invention was created considering the abovementioned circumstances, and its purpose is to provide a sample container in which a specimen, etc., can be easily enclosed in a container, and which has a stable rupture mode and can reliably supply immersion fluid to a storage container.

### Means for Solving the Problems

A sample container according to the present invention created to address the problems noted above comprises: a storage container provided with a storage apparatus for holding a sample; and a reservoir container equipped with a reservoir apparatus for storing an immersion fluid in which to immerse the sample and a sealing film for sealing an opening section of the reservoir apparatus. The storage container is provided with an inner coupling section that surrounds the opening section of the reservoir apparatus and guides the immersion fluid released from the reservoir apparatus to the storage apparatus, and is provided with an unsealing blade that faces an inner edge section of the opening section of the reservoir apparatus. The reservoir container is provided with an outer coupling section that couples with the inner coupling section of the storage container using a screw pair. The screw pair is provided with an impeding region that suppresses progression of tightening before contact of the sealing film and the unsealing blade. The communication between the reservoir apparatus and the storage apparatus is ensured by entry of the unsealing blade of the storage apparatus to inside the reservoir apparatus by tightening beyond the impeding region of the screw pair and rupturing the sealing film.

The sample container may adopt a configuration in which unsealing blades are provided at equal cycles at the opening section of the storage apparatus, and the screw pair is provided with a termination region which stops rotation at rotation less than the intermittent angle of the unsealing blade after contact between the sealing film and the unsealing blade.

Here, the intermittent angle of the unsealing blade is the phase (angle) range of the portion of the opening section of the storage apparatus equipped with the unsealing blade, within the range of one cycle of the unsealing blade arrangement, that is set so there is no contact with the sealing film, or at the point there is contact, it stops without rupturing the sealing film, so as not to enter inside the reservoir apparatus.

The sealing film adopts a constitution made from a material that is separated by pressure deformation caused by the unsealing blade.

For example, a constitution may be adopted that is provided with a shearing means that uses the inner edge of the opening section of the reservoir apparatus as a die, and uses the unsealing blade of the opening section of the storage apparatus as a punch, and the sealing film can be made from a material that is separated (cut) by shearing deformation caused by the shearing means.

It is also possible to adopt a constitution that gives water repellency to the inner surface of the reservoir apparatus or the inner surface of the sealing film by forming a fluorine-based liquid-repellent resist thin film , etc., or a constitution in which the unsealing blade is provided with a groove or slit that guides the immersion fluid released from the reservoir apparatus to the storage apparatus.

### Effect of the Invention

According to the sample container of the present invention, by opening a screw cap and enclosing a sample in the sample container, and by forcefully performing a tightening operation to close the screw cap, the specimen, etc., is easily enclosed in the container without exposing the immersion fluid enclosed in the storage part to the outside, and it is possible to simultaneously reliably supply the immersion fluid into the storage apparatus into which the specimen, etc., has been input. That makes it possible to avoid the risk of forgetting the operation of supplying the immersion fluid and damaging the sample.

In the screw pair of the sample container according to the present invention, by providing an impeding region that suppresses progression of tightening before contact of the sealing film and the unsealing blade, and a termination region that stops the rotation at a rotation of less than the intermittent angle of the unsealing blade after contact of the sealing film and the unsealing blade, it is possible to uniformly control the shape and position of the rupturing section that connects the reservoir apparatus and the storage apparatus, and possible to avoid the risk of damage to the sample of the specimen, etc., by foreign matter contamination due to connection failure or rupture of the sealing film.

By adopting a constitution that places the unsealing blade for rupturing the sealing film that seals the revoir apparatus at the end edge of the side wall opening section of the storage apparatus, there is no obstruction inside the storage apparatus, so it is easy to take the sample in and out, and possible to prevent adverse effects on the sample such as scratches.

By adopting an item made from a material that is separated using pressure deformation by the unsealing blade for the sealing film, it is possible to more reliably control the shape and position of the rupturing section that allows communication.

Furthermore, for example, by adopting a constitution that provides a shearing means that that uses the inner edge of the opening section of the reservoir apparatus as a die, and uses the unsealing blade of the opening section of the storage apparatus as a punch, and in which the sealing film is made of a material that is separated (cut) by shearing deformation using the shearing means, accuracy is improved.

By adopting a constitution that gives water repellency to the inner surface of the reservoir apparatus or the inner surface of the sealing film according to the viscosity, properties, etc., of the immersion fluid, or a constitution that provides a groove or slit that guides the immersion fluid released from the reservoir apparatus to the storage apparatus, it is possible to avoid trouble such as spilling of residual immersion fluid inside the reservoir apparatus when taking the sample out from the storage apparatus.

### Brief Description of the Drawings

[FIG. 1] FIG. 1 is a vertical cross section illustrating the (A) unused state and (B) used state of a sample container according to the present invention.
[FIG. 2] FIG. 2 is a vertical cross section showing an example of a screw pair illustrating the (A) unused state and (B) used state of the sample container according to the present invention.
[FIG. 3] FIG. 3 is (A) a plan view and (B) a bottom view of a storage container of the sample container according to the present invention.
[FIG. 4] FIG. 4 is (A) a vertical cross section of a reservoir container and (B) a vertical cross section with a portion cut away of the reservoir apparatus of the sample container according to the present invention.
[FIG. 5] FIG. 5 is (A) a plan view and (B) a bottom view of the reservoir container of the sample container according to the present invention.
[FIG. 6] FIG. 6 is a side view showing an example of (A) an open state, (B) an unused state, and (C) a used state of the sample container according to the present invention.
[FIG. 7] FIG. 7 is a vertical cross section showing an example of (A) the unused state and (B) the used state of the sample container according to the present invention.
[FIG. 8] FIG. 8 is a vertical cross section showing an example of the screw pair illustrating (A) the unused state and (B) the used state of the sample container according to the present invention.
[FIG. 9] FIG. 9 is (A) a plan view and (B) a bottom view of the storage container of the sample container according to the present invention.
[FIG. 10] FIG. 10 is (A) a vertical cross section and (B) a vertical cross section with a portion cut away of the reservoir apparatus of the reservoir container of the sample container according to the present invention.
[FIG. 11] FIG. 11 is (A) a plan view and (B) a bottom view of the reservoir container of the sample container according to the present invention.
[FIG. 12] FIG. 12 is a side view illustrating (A) the open state, (B) the unused state, and (C) the used state of the sample container according to the present invention.
[FIG. 13] FIG. 13 is (A) a plan view, (B) a bottom view, and (C) a perspective view of the reservoir container of the sample container according to the present invention.

### Modes for Carrying Out the Invention

Hereafter, an embodiment of a sample container is described in detail based on the drawings.

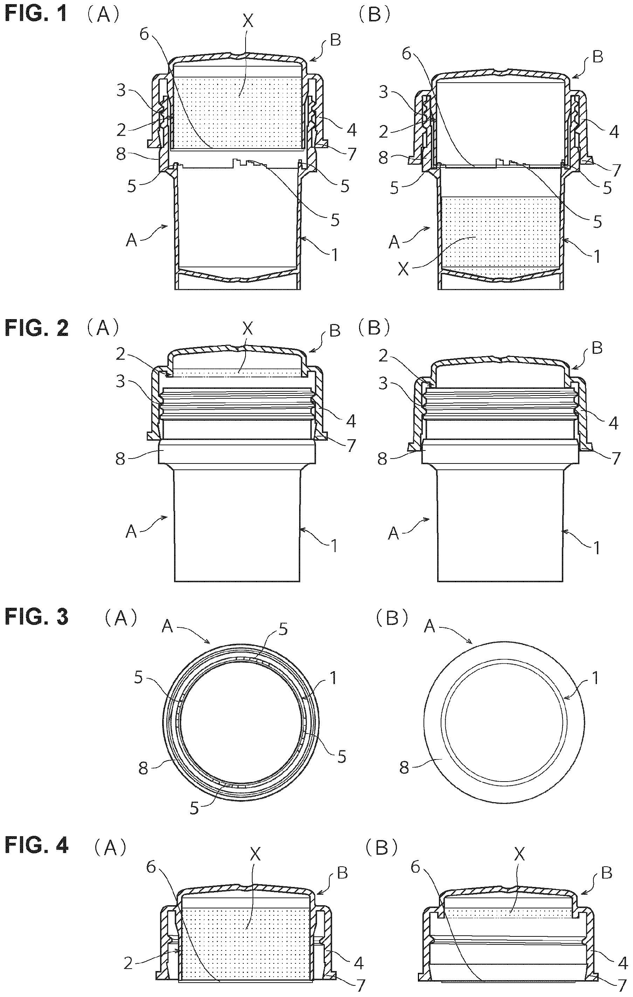

The sample container shown in FIG. 1 comprises a storage container A provided with a storage apparatus 1 for holding a sample, and a reservoir container B provided with a reservoir apparatus 2 for storing an immersion fluid X in which the sample is immersed.

The storage container A comprises: the storage apparatus 1; an inner coupling section 3 that surrounds an opening section of the reservoir apparatus 2 to receive the immersion fluid X released from the reservoir apparatus 2 and guide it to the storage apparatus 1; and an unsealing blade 5 facing the inner edge section of the opening section of the reservoir apparatus 2 (follows the inner edge section).

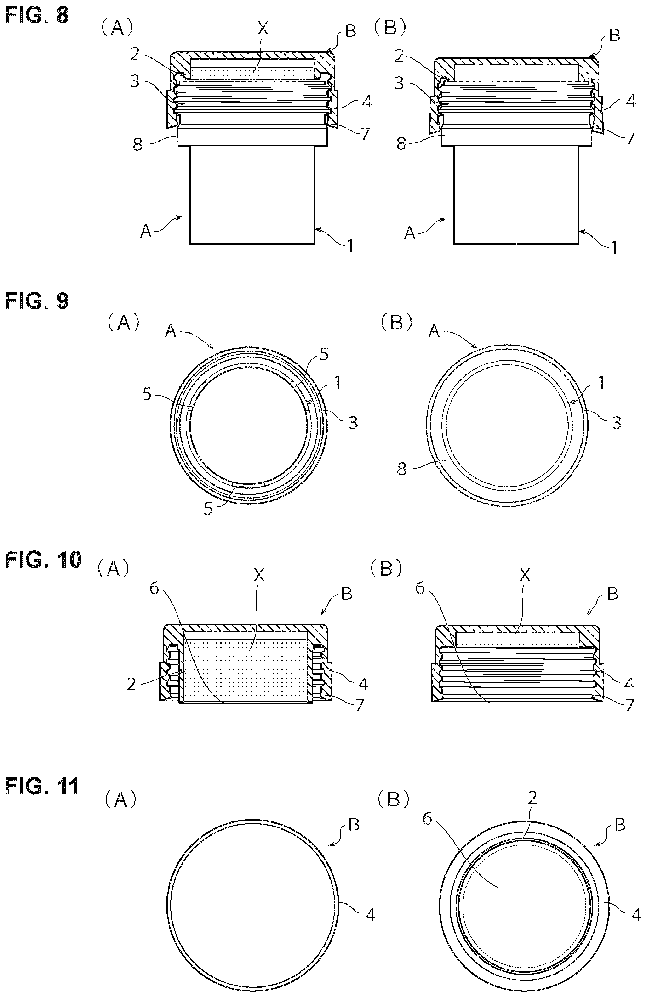

The storage apparatus 1 of this example is a bottomed cylinder-shaped container made of synthetic resin, and the inner coupling section 3 is a concentric cylinder-shaped part that continues from the opening section of the storage apparatus 1 to the upper part as an outer cylinder, and a male screw of a screw pair is drilled on the outside surface (see FIG. 2 or FIG. 8).

The reservoir container B comprises the reservoir apparatus 2, and an outer coupling section 4 surrounding the inner coupling section 3.

The reservoir apparatus 2 of this example is a bottomed cylinder-shaped synthetic resin container, and the outer coupling section 4 is a concentric cylinder-shaped part that bulges to the outside of the reservoir apparatus 2 from a base of the reservoir apparatus 2 and is continuous as an outer cylinder along a side wall of the reservoir apparatus 2, and a female thread of the screw pair (screwed together with the male screw of the inner coupling section 3) is drilled on the inside surface.

The reservoir apparatus 2 is filled with a predetermined amount of the immersion fluid X, and the opening section of the reservoir apparatus 2 is sealed by a sealing film 6 using heat sealing, etc.

The sealing film 6 preferably uses a material that is highly easy to open such as polypropylene film, a material that is separated by shearing deformation using the shearing means such as a metal foil, or a material that layers these.

Furthermore, to not leave behind immersion fluid X, which can spill during unsealing, inside the reservoir apparatus 2, it is preferable to give water repellency to the inner surface of the sealing film 6 and the inner surface of the reservoir apparatus 2, and for example, it is possible to use a method of applying a thin film of a fluorine-based polymer, etc., as a liquid repellent resist thin film.

In the sample container, by the inner edge of the vertically cut opening section of the reservoir apparatus 2 being formed as a blade of the die, and the outer edge of the unsealing blade 5 protruding to the front from the opening section (end edge of the outer wall) of the storage apparatus 1 being formed as a blade of the punch, it is possible to constitute the shearing means for rupturing the sealing film 6 by tightening the screw pair.

The unsealing blade 5 can be provided as one or a plurality according to the pitch of the screw pair, etc., and when a plurality is provided, it is preferable to arrange them at equal cycles (equal angle intervals in this example) along the entire circumference of the opening section of the storage apparatus 1.

In the example shown in FIG. 1 (hereafter noted as "Embodiment 1"), each unsealing blade 5 is equipped with a plurality of punches provided with a difference in height along the slope of the male screw, and the punches equipped in each unsealing blade 5 are arranged in a right-angled triangular shape sandwiching a slit extending approximately the full length of each. The slit is a notch for guiding the immersion fluid X released from the reservoir apparatus 2 after unsealing to the storage apparatus 1, but it is also possible to adopt a constitution that replaces the slit with a groove.

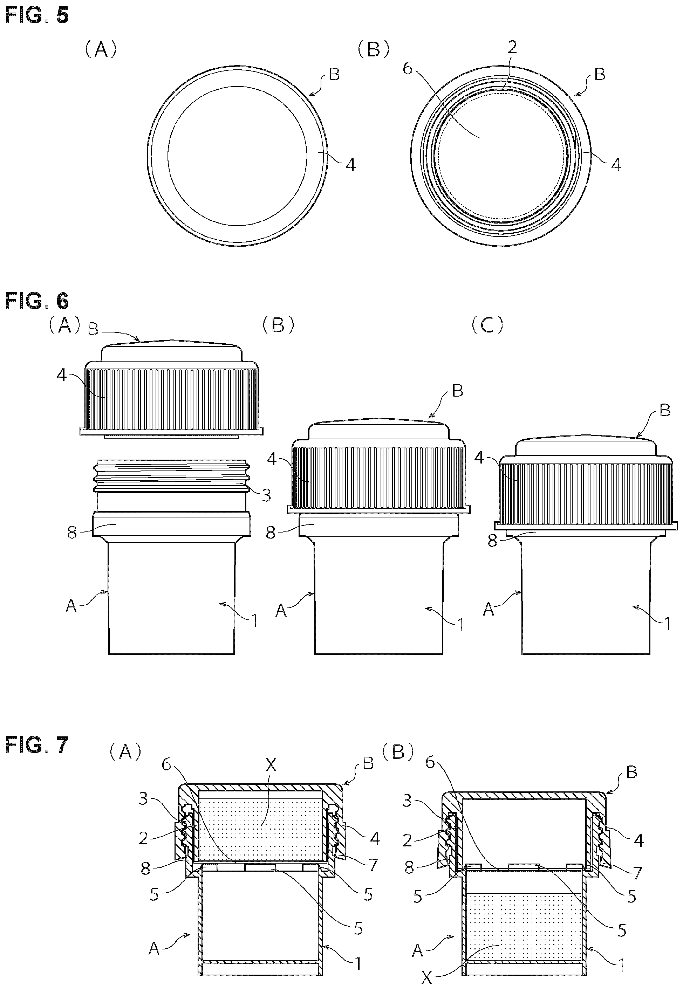

In the example shown in FIG. 7 (hereafter noted as "Embodiment 2"), each of the unsealing blades 5 is formed in a rectangular shape having a uniform height. In this example, three unsealing blades 5 are provided at 120-degree intervals (120-degree cycles). Even in this example, it is possible to provide a single or a plurality of slits or grooves for each unsealing blade 5.

The shearing means, according to the constitution noted above, pressurizes the sealing film 6 in a mutually opposing direction to displace it along the target cutting surface, thereby breaking the tissue bond at the cutting surface and separating the rupture site.

When using a material with high elasticity, etc., as the sealing film 6, instead of a constitution as a shearing means, it is possible to use a sharp cutting blade as the unsealing blade 5 (see FIG. 7).

By the male screw of the storage container A and the female thread of the reservoir container B being screwed together, the opening section of the storage apparatus 1 and the opening section of the reservoir apparatus 2 are sealed.

The screw pair comprises an impeding region in which the progression of tightening is suppressed before contact of the sealing film 6 and the unsealing blade 5, and a termination region that stops rotation at rotation of an angle that ensures sufficient communication between the storage apparatus 1 and the reservoir apparatus 2 after contact between the sealing film 6 and the unsealing blade 5, and at which part or all of the sealing film 6 does not detach from the opening section of the reservoir apparatus 2 (e.g. rotation less than the intermittent angle of the unsealing blade 5).

The impeding region is a region in which in the combination of the tip inner surface of the outer coupling section 4 and the base outer surface of the inner coupling section 3, or the combination of the base inner surface of the outer coupling section 4 and the tip of the inner coupling section 3, etc., a mutual interference shape is given to provide tightening resistance or loosening resistance to the extent that advance and retreat or relative rotation by the screw pair cannot be done without intentionally performing a screwing operation (see FIG. 1 (A), FIG. 2 (A) or FIG. 6 (B), or FIG 7 (A), FIG. 8 (A), or FIG. 12 (B)).

For example, according to FIG. 2 (A) or FIG. 8 (A), before contact between the sealing film 6 and the unsealing blade 5, a projecting part 7 provided on a part or all of the tip inner surface on the outer coupling section 4 and a protrusion 8 formed in a flange shape on the base outer surface of the inner coupling section 3 interfere with each other, forming the impeding region in which progression of mutual tightening is suppressed. When further tightening is done, and the projecting part 7 provided at the tip of the outer coupling section 4 surpasses the protrusion 8, resistance decreases (see FIG. 2 (B) or FIG. 8 (B)), but when further tightening is done, after the sealing film 6 and the unsealing blade 5 contact each other, the abutment part formed on the base inner surface of the outer coupling section 4 and the tip of the inner coupling section 3 interfere at a rotation of less than the intermittent angle of the unsealing blade 5 (less than 60 degrees in the example in FIG. 3 (preferably less than 40 degrees), and less than 90 degrees in the example in FIG. 9 (preferably less than 60 degrees)), forming the termination region in which mutual rotation is stopped (see FIG. 1 (B) or FIG. 7 (B)).

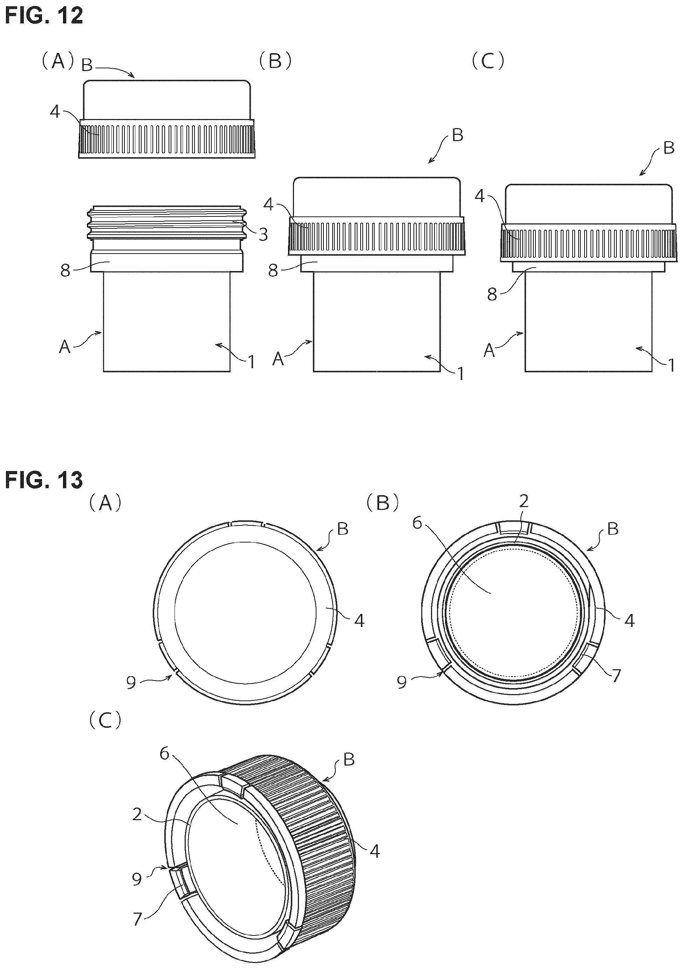

When the projecting part 7 is provided on part of the tip inner surface of the outer coupling section 4, it is possible to adopt a constitution that interposes a notch 9 at the boundary between a region equipped with the projecting part 7 and a region not equipped with it (see FIG. 13).

Instead of the abovementioned constitution, the termination region may also adopt a constitution in which the tip of the outer coupling section 4 and high protrusions formed in a flange shape on the base outer surface of the inner coupling section 3 interfere with each other and stop mutual rotation (not illustrated).

When unused and being transported, the sample container constituted as described above is placed in a position (hereafter noted as "standby position") that achieves mutual interference of the tip inner surface of the outer coupling section 4 of the reservoir container B which serves as the lid of the storage container A and the protrusion 8 formed in a flange shape on the base outer surface of the inner coupling section 3 (see FIG. 6 (B) or FIG. 12 (B)).

In the standby position, the tip of the outer coupling section 4 is slightly pushed apart, and is integrated in a state that suppresses the progression of tightening. At this time, the reservoir container B is pushed back in the loosening direction, so that its female thread and the male screw of the storage container A come into close contact, forming a stable coupling state without rattling or rotating with each other.

In this pre-use state, the immersion fluid X is enclosed in the reservoir apparatus 2 in the standby position, and the sealing film 6 seals the opening section of the reservoir apparatus 2 in a state in close contact with the end edge of the reservoir apparatus 2.

The work of inputting the sample into the storage apparatus 1 is achieved by loosening the reservoir container B and removing it from the storage container A (see FIG. 6 (A) or FIG. 12 (A)), putting the sample into the storage apparatus 1 and again coupling the reservoir container B to the storage container A. When the reservoir container B is tightened into the storage container A, the standby position is reached again, conveying to the hand the sense of suppression of the progression of tightening, but tightening continues forcefully without stopping tightening at this standby position, and when the termination region is reached, the abutment part formed on the base inner surface of the outer coupling section 4 and the tip of the inner coupling section 3 interfere with each other, and the hand senses stopping of rotation, and tightening ends_(see FIG. 6 (C) or FIG. 12 (C)).

At this time, by the reservoir container B being pushed back in the loosening direction, the female thread and the male screw of the storage container A are in close contact, forming a stable coupling state with no mutual rattling or rotation, and a state with a relatively high level of airtightness.

At this time, inside the sample container, the sealing film 6 is ruptured at a set trajectory by the tightening operation between the standby position and the termination region, and there is pressing to the inside of the reservoir apparatus 2 by pressure by the unsealing blade 5 near the rupture site, ensuring communication between the reservoir apparatus 2 and the storage apparatus 1.

At this time, in Embodiment 1, the pressing action or shearing action is applied by pressuring close to a point or short line, while in Embodiment 2, the pressing action or shearing action is applied by pressing close to a line.

With the condition that the reservoir container B be on the upper part of the storage container A, the immersion fluid X of the reservoir container B flows to inside the storage apparatus 1 from a hole that occurs in the rupture site of the sealing film 6. When a slit or groove is provided in the unsealing blade 5, the immersion fluid X also flows in via the slit or the groove.

Furthermore, when water repellency has been given to the inner surface of the reservoir apparatus 2 or the inner surface of the sealing film 6, the immersion fluid X inside the reservoir apparatus 2 falls into the storage apparatus 1 without delay.

### Explanation of Codes

- A: Storage container
- B: Reservoir container
- X: Immersion fluid
- 1: Storage apparatus
- 2: Reservoir apparatus
- 3: Inner coupling section
- 4: Outer coupling section
- 5: Unsealing blade
- 6: Sealing film
- 7: Projecting part
- 8: Protrusion
- 9: Notch

## Claims

1. A sample container, comprising:
a storage container provided with a storage apparatus for holding a sample; and
a reservoir container equipped with a reservoir apparatus for storing an immersion fluid in which to immerse the sample and a sealing film for sealing an opening section of the reservoir apparatus, **characterized in that**
the storage container is provided with an inner coupling section that surrounds the opening section of the reservoir apparatus and guides the immersion fluid released from the reservoir apparatus to the storage apparatus, and is provided with an unsealing blade that faces an inner edge section of the opening section of the reservoir apparatus,
the reservoir container is provided with an outer coupling section that couples with the inner coupling section of the storage container using a screw pair,
the screw pair is provided with an impeding region that suppresses progression of tightening before contact of the sealing film and the unsealing blade, and
the communication between the reservoir apparatus and the storage apparatus is ensured by entry of the unsealing blade of the storage apparatus to inside the reservoir apparatus by tightening beyond the impeding region of the screw pair and rupturing the sealing film.

2. A sample container, comprising:
a storage container provided with a storage apparatus for holding a sample; and
a reservoir container equipped with a reservoir apparatus for storing an immersion fluid in which to immerse the sample and a sealing film for sealing an opening section of the reservoir apparatus, **characterized in that**
the storage container is provided with an inner coupling section that surrounds the opening section of the reservoir apparatus and guides the immersion fluid released from the reservoir apparatus to the storage apparatus, and is provided with unsealing blades facing the inner edge section of the opening section of the reservoir apparatus, at equal cycles on the opening section of the storage apparatus,
the reservoir container is provided with an outer coupling section that couples with the inner coupling section of the storage container using a screw pair,
the screw pair is provided with an impeding region that suppresses progression of tightening before contact of the sealing film and the unsealing blade, and a termination region that stops the rotation at a rotation of less than the intermittent angle of the unsealing blade after contact of the sealing film and the unsealing blade,
the communication between the reservoir apparatus and the storage apparatus is ensured by entry of the unsealing blade of the storage apparatus to inside the reservoir apparatus by tightening beyond the impeding region of the screw pair and rupturing the sealing film.

3. The sample container according to claim 1, **characterized in that** water repellency is given to the inner surface of the reservoir apparatus or the inner surface of the sealing film.

4. The sample container according to claim 1, comprising a shearing means that uses the inner edge of the opening section of the reservoir apparatus as a die, and uses the unsealing blade of the opening section of the storage apparatus as a punch,
**characterized in that** the sealing film can be made from a material that is separated by shearing deformation caused by the shearing means.

5. The sample container according to any of claims 1 to 4, **characterized in that** the unsealing blade is provided with a groove or slit that guides the immersion fluid released from the reservoir apparatus to the storage apparatus.
